## Europäisches Patentamt
## European Patent Office
## Office européen des brevets

(19)

(11) Publication number: **0 039 962**

**B1**

(12)

# EUROPEAN PATENT SPECIFICATION

(45) Date of publication of patent specification: **02.03.83**

(51) Int. Cl.³: **C 12 P 19/06, C 08 B 37/00**

(21) Application number: **81200414.1**

(22) Date of filing: **10.04.81**

(54) Clarification of polysaccharide-containing fermentation products.

(30) Priority: **08.05.80 US 147812**

(43) Date of publication of application:
**18.11.81 Bulletin 81/46**

(45) Publication of the grant of the patent:
**02.03.83 Bulletin 83/9**

(84) Designated Contracting States:
**CH DE GB LI NL**

(56) References cited:
FR - A - 2 264 077
US - A - 3 969 189
US - A - 4 119 491
US - A - 4 165 257
CHEMICAL ABSTRACTS, vol. 91, no. 14, April 6, 1981, page 107, abstract 105255f Columbus, Ohio, USA
GRIFFITH, W. L. et al. "Modification of biopolymer solution properties by endoglucanohydrolases"

(73) Proprietor: **SHELL INTERNATIONALE RESEARCH MAATSCHAPPIJ B.V.**
Carel van Bylandtlaan 30
NL-2596 HR Den Haag (NL)

(72) Inventor: **Rader, William Ernest**
4901 Dale Road
Modesto California 95350 (US)
Inventor: **Wong, Jane Chang**
466 Shire Way
Modesto California 95356 (US)

(74) Representative: **Rogers, Roy Charles et al,**
4 York Road
London SE1 7NA (GB)

Courier Press, Leamington Spa, England

# Clarification of polysaccharide-containing fermentation products

This invention relates to an improved method of clarifying aqueous polysaccharide solutions obtained by fermentation of a nutrient medium with a xanthan-producing microorganism. More particularly, this invention is directed to an improved enzyme treatment process for degrading particulate material in such polysaccharide-containing solutions wherein a complex enzyme system is employed at a lower concentration and milder treatment conditions than heretofore possible to afford a biopolymer product having superior handling properties.

Polysaccharide polymers produced by xanthomonas fermentation of carbohydrates have established utility as viscosity enhancers in enhancement of oil recovery. In this application the hydrophilic polysaccharides or xanthan gums are added at a low concentration to water flooding operations to increase the viscosity of the aqueous system and thereby bring about more efficient, piston-type, displacement of the oil from the reservoir rock. These microbial polysaccharides appear to be particularly well suited for this polymer flooding application in that they exhibit, in aqueous systems, a high viscosity at low concentration that is stable to the high shear rates encountered at the point of injection into the oil-bearing formation and which is largely insensitive to high salt concentration found in formation waters.

While the outlook for the use of microbial polysaccharides in enhanced oil recovery would appear to be promising, certain problems have been encountered in practice which have served to reduce the previous estimates of polymer requirements for this end use application. Our area of difficulty relates to the insoluble impurities present in industrial grades of these xanthan gums. In the typical commercial production of polysaccharides by xanthomonas fermentation, the high viscosity of the fermentation broth precludes complete separation of insoluble material such as cellular debris and nonviable bacteria from the polysaccharide-containing broth. As a result, commercial grades of these microbial polysaccharides, i.e. xanthan gums, contain solids which do not dissolve when the xanthan gum is placed in dilute aqueous solution such as that required for polymer flooding in enhanced oil recovery. The presence of these particulate solids in the polysaccharide solution presents considerable difficulty in field application of the polymer flood because they can cause plugging of the rock face and injection water filters. Previous attempts to overcome this plugging problem have included caustic treatment of the polysaccharide solution and subsequent flocculation of the solids, and enzyme treatment to bring about chemical decomposition of the solid material in the polysaccharide solution prior to use. Although the enzymatic treatment has definite advantages over the caustic addition and flocculation technique under practical use conditions encountered in polymer flooding operations, it has hitherto required either the use of treatment at elevated temperatures or extended aging times to be effective.

An enzyme treatment has now been found which degrades water insoluble material, e.g. cellular debris, in aqueous polysaccharide solutions derived from xanthomonas fermentation of carbohydrates without the need for high temperature and/or long residence time aging. This enzyme treatment utilizes a complex enzyme having cell-lytic, $\beta$-1,3-glucanase and protease activity which produces effectively complete degradation of cellular debris in the polysaccharide solution with very low levels of the enzyme complex, e.g. 100 ppm or less, without significant adverse effect on the viscosity of the resultant polysaccharide solution. Further, the efficacy of this enzyme complex treatment is such that treatment of these polysaccharide solutions containing cellular debris can be typically carried out at ambient temperatures (about 25°C) for residence times of less than 15 min to afford a polysaccharide sufficiently free of insoluble solid material that it is suitable for use in enhanced oil recovery.

Accordingly, the present invention provides a process for degrading water insoluble matter present in an aqueous polysaccharide solution derived from xanthomonas fermentation of carbohydrates, which comprises contacting the aqueous polysaccharide solution with a complex enzyme having cell-lytic, $\beta$-1,3-glucanase and protease activity, said complex enzyme being obtained from *Pellicularia filamentosa* and/or *Pellicularia sasakii*. Also within the scope of the present invention are solid formulations containing xanthan gum and the enzyme complex. These solid formulations are of special advantage in that they facilitate carrying out of the process of the invention in a simple single step operation at, for example, the field location where the enhanced oil recovery is to be carried out.

The enzyme employed in the process of the invention is an enzyme complex having particular ability to dissolve the cells of microorganisms in the genus xanthomonas. These complex enzymes, which are produced by known strains of the genus Pellicularia, exhibit activities including cell-lytic activity, $\beta$-1,3-glucanase and protease activity. Suitable enzyme complexes are those produced by the species *Pellicularia sasakii* and *Pellicularia filamentosa* as described in U.S. Patent 3,969,189 to Kobayashi et al. According to the disclosure of this U.S. Patent, which is herewith incorporated by reference, the complex enzymes are produced by cultivating the aforementioned species of the genus Pellicularia in a culture medium containing assimilable carbon sources, such as starch, glucose or wheat bran, and assimilable nitrogen sources, such as ammonium salts, yeast extract or defatted soybean meal, to produce in the medium an accumulation of the enzyme which can be isolated and purified by conventional techniques. Preferably, the cultivation is carried out under aerobic conditions at 20—35°C for 2 to 10 days. In accordance with the invention, a preferred source of the enzyme complex is the *Pelli-*

*cularia filamentosa vir. sasakii* strain isolated from a lesion of the black scurf infection in potato plant and deposited with the American type culture collection under ATCC No. 20365 (This strain has been earlier designated erroneously as ATCC No. 14701). This preferred strain of the genus Pellicularia is used to produce the enzyme complex available commercially under the trade name "Kitalase" from Kumiai Chemical Industry Co. Ltd., Tokyo, Japan. It is believed that the complex enzymes obtained from the aforementioned strains of the genus Pellicularia are a mixture of component enzymes each having a molecular weight of at least 50,000 which together are known to possess multiple enzymatic activities including cellulose, carboxymethyl cellulose, glucanase, chitanase, protease, hemi-cellulose and amylase activities. These several enzymes with their associated activities are believed to interact to afford the high activity in lysing microorganism cells observed for the enzyme complex.

The polysaccharide employed in the process of the invention is a hydrophilic colloid obtained by fermentation of a suitable nutrient medium with microorganisms of the genus Xanthomonas. These xanthum gums and their preparation are well known in the art, see for example U.S. Patents 3,659,026, 3,801,502 and 3,966,618. Because of commercial availability, it is preferred to use the polysaccharide produced by *xanthomonas campestris* ATCC 13951 although other conventional xanthomonas bacteria such as *xanthomonas phaseoli, xanthomonas carotae, xanthomonas begoniae, xanthomonas meanae, xanthomonas malvacearum, xanthomonas vesicatoria, xanthomonas papavericola, xanthomonas incanae* and *xanthomonas translucens* may be employed. The polysaccharide may be used in the form of the fermentation broth or medium, that is the aqueous nutrient medium containing undissolved solids and cell debris in addition to the water soluble xanthan gum, or as an aqueous solution obtained by dissolving solid xanthan gum, isolated from the broth by known procedures, in an aqueous media. A xanthan gum suitable for use in the process of the invention is that produced by the Kelco Company, San Diego, California, under the trade name KELZAN MF. This commerical product although having technically useful viscosity properties, contains cell debris which can advantageously be degraded by the process according to the invention to increase its suitability for enhanced oil recovery.

The process according to the invention is carried out by adding the complex enzyme to an aqueous solution of the polysaccharide containing the insoluble fermentation cell debris and allowing the mixture to stand, with or without agitation, for a sufficient time to allow substantially complete degradation and dissolution of the cellular material by enzymatic action. The aqueous solution employed suitably contains from 1.0 to 0.03% by weight of polysaccharide (xanthan gum) with concentrations in the range of 0.8 to 0.5% by weight being preferred. As noted above, this aqueous polysaccharide solution may be the xanthomonas fermentation broth itself (diluted and undiluted) or it may be prepared by dissolving a measured amount of solid xanthan gum, isolated from the fermentation broth, in aqueous media. The concentration employed of the complex enzyme having cell-lytic, $\beta$-1,3-glucanase and protease activity will depend on the concentration of xanthum gum used and the extent to which it is contaminated with insoluble cellular debris. For typical commercial grade xanthan gums, e.g. KELZAN MF, the complex enzyme is suitably added to the aqueous polysaccharide solution in an amount sufficent to give a treated solution containing from 10 to 100 ppm of the enzyme complex. Preferably, the enzyme is employed at a concentration of 20 to 30 ppm in the aqueous solution containing the polysaccharide and insoluble cellular debris. The amount of complex enzyme to be added to the aqueous polysaccharide solution may alternatively be determined on the basis of units of cell wall-lysing potency or activity provided in the solution as determined by the procedure described in column 5, lines 24—41, of the aforementioned U.S. Patent 3,969,189. Based on enzymatic activity determined by this test, the process of the present invention is suitably carried out using from 60 to 250, preferably from 500 to 300, cell wall-lysing units of complex enzyme per gram of xanthan gum.

As noted above, the process according to the invention may be carried out with or without agitation; however, it is preferred to maintain sufficient stirring action in the treated solution to avoid settling of solids and to insure a uniform concentration of enzyme throughout the polysaccharide solution. With the complex enzymes of the invention the cell degradation and solution clarifying reaction will proceed at a satisfactory rate at ambient temperatures (e.g. 25°C) without the addition of heat normally being necessary to drive the reaction. Depending on the actual climatic conditions at the site where the polysaccharide solution clarification takes place, e.g. the well site, some warming of the solution may be needed to obtain the optimum reaction rate. Suitably, the aqueous solution containing polysaccharide, insoluble cellular debris and the complex enzyme is maintained at from 20 to 35°C during the reaction period. Preferably, the temperature is held between 25 and 30°C during the period of time when the cell wall degradation reaction is taking place. For optimum cell degradation activity, the aqueous polysaccharide solution is suitably maintained at a pH of between 2 and 10 and preferably at a pH of 3.4 to 8.0. Under the conditions described above, substantially complete (up to 98%) degradation the insoluble cellular material can be obtained in from 0.25 to 0.50 hours. At reaction temperatures above 25°C the optimum reaction time will typically not exceed 0.25 hours, particularly when the pH is maintained at between 5.0 and 7.0.

The process according to the invention may be carried out batchwise or continuously by adding the complex enzyme to a reservoir or tank containing the aqueous polysaccharide, as diluted or undiluted fermentation broth or aqueous solution made up from the isolated xanthan gum. If the process is carried out continuously the reaction vessel or tank should be sized sufficiently and the rate of enzyme and polysaccharide addition should be such that the aqueous polysaccharide solution containing solid cellular debris is given sufficient residence time in the presence of an appropriate concentration of complex enzyme to afford the desired degree of cell degradation. Upon completion of the reaction period, the enzyme-treated polysaccharide solution may be passed with or without aqueous dilution directly to the oil recovery operation, or alternatively, all or a portion of the water may be removed to afford a concentrated solution or solid polysaccharide which can be transported and/or stored for subsequent use. In oil recovery, the treated solution, concentrate and/or solid is diluted with additional water or other aqueous media to a polysaccharide concentration of 250 to 750 ppm, preferably, 300 to 350 ppm.

In another embodiment, the present invention also includes solid formulations containing xanthan gum and the enzyme complex produced by *Pellicularia sasakii* and/or *Pellicularia filamentosa*, preferably ATCC 20365, which can be conveniently employed under field conditions in enhanced oil recovery. Suitable solid formulations contain 90 to 99.5% by weight xanthan gum, 0.3 to 0.5% by weight complex enzyme having cell-lytic, $\beta$-1,3-glucanase and protease activity and from 0 to 10% by weight to a solid extender or inert diluent. These solid formulations can be added directly to water or other aqueous media at the field location where the enhanced oil recovery is being carried out, thereby eliminating the need for separate addition of the enzyme and xanthan gum.

## Example I

Comparative experiments were carried out to determine the relative effectiveness of a complex enzyme according to the invention (KITALASE, Kumiai Chemical Industry Co. Ltd) and an alkaline protease enzyme of the prior art (ALCALASE, Novo Industries A/S) in removing insoluble cellular debris from a xanthomonas derived polysaccharide. The polysaccharide or xanthan gum used in these experiments was KELZAN MF (Kelco Company, San Diego, California) a commercial xanthan gum produced by fermentation of a nutrient medium with *xanthomonas campestris*. Measured amounts of the xanthan gum and respective enzymes were added (the xanthan gum in the form of a dry powder) to synthetic USBR water a) maintained at 24°C in a Lightnin "mixer" to form a concentrated aqueous solution containing 6000 ppm of xanthan gum and 25 ppm of the enzyme. After addition of the xanthan gum and enzyme the mixture was stirred for 15 minutes at 24°C. Subsequently, the concentrated solution was diluted with additional USBR water to afford an aqueous solution containing 300 ppm of the xanthan gum. The experiments with each enzyme were carried out in duplicate and in the second experiment in each case the enzyme treated xanthan gum solution was subject to shearing in a shear plate mixer using a 0.38 mm orifice with a pressure differential of 35 kg/cm² prior to dilution with additional USBR water. The effectiveness of the enzyme treatments in removing solid cellular debris from the xanthan gum solution was determined both by optical density and by filterability. In this respect, filterability was determined by passing the treated and diluted xanthan gum solutions through a 1.2 $\mu$ Millipore filter and measuring the quantity of liquid filtrate which collects in a 5 minute period at a positive pressure of 1.4 kg/cm². Viscosity of the xanthan gum solution was also measured with a Brookfield microviscometer using a UL adapter. A control comparison was carried out using no enzyme. The results of the comparative tests are given below in Table I.

a) Synthetic USBR water (pH—8.1) had the following salt and mineral composition:

| | |
|---|---|
| Mg SO₄ 7H₂O | 10 ppm |
| Ca SO₄ | 20 ppm |
| Na Cl | 100 ppm |
| Na HCO₃ | 85 ppm |
| Na SO₃ | 50 ppm |

**0 039 962**

TABLE I

Enzyme Treated Solution

| Enzyme Treatment | Filterability Flow Rate, ml/5 min. | Turbidity Optical Density at 660 rm | Polymer Content | Viscosity cp at 38°C | | |
|---|---|---|---|---|---|---|
| | | | | 7.3 Sec-1 | 14.0 Sec-1 | 30.5 Sec-1 |
| Alcalase | | | | | | |
| Sheared | 1090 | 0.020 | 294 | 3.7 | 3.25 | 2.68 |
| Not Sheared | 513 | 0.025 | 293 | 4.0 | 3.25 | 2.74 |
| Kitalase | | | | | | |
| Sheared | 1200 | 0.010 | 294 | 4.0 | 3.10 | 2.68 |
| Not Sheared | 552 | 0.012 | 288 | 3.8 | 3.13 | 2.60 |
| Control (no enzyme treatment) | | | | | | |
| Sheared | 190 | — | 300 | 3.8 | 3.25 | 2.88 |
| Not Sheared | 130 | — | 300 | 3.5 | 3.25 | 2.88 |

It is apparent from the table above that treatment with Kitalase, a complex enzyme according to the invention, gives the polysaccharide solution better filterability both before and after shearing than is obtained by treating the polysaccharide solution in the identical manner with Alcalase, an alkaline protease enzyme of the prior art. Further the clarity of the Kitalase preparation is better as measured by optical density at 660 rm. The viscosities of the polysaccharide solutions treated with both enzyme compositions were essentially the same as obtained with the untreated control solution, either before or after shearing.

Example II

Using the procedure given in Example I, in aqueous polysaccharide soltuion containing 6000 ppm of xanthan gum (KELZAN MF) was prepared in USBR water and treated with varying amounts of Kitalase, a complex enzyme according to the invention, and the treated solutions were diluted with additional USBR water to the 300 ppm xanthan gum concentration level for determination of properties. In this case, the properties determined for each enzyme treatment level included the viscosity and filterability tests described in Example I and a determination of the number of dead bacterial bodies in the treated polysaccharide solutions. The results of the tests, including the enzyme treatment levels, are given in Table II below.

5

TABLE II

| Amount of Kitalase (ppm) | Flow Rate of 300 ppm Dilution ml/5 min Thru 1.2 $\mu$ [a] | No. of Bacteria/ml 300 ppm Dilution | Viscosity at 38°C (cp) | | | |
|---|---|---|---|---|---|---|
| | | | 7.3 Sec$^{-1}$ | 14.6 Sec$^{-1}$ | 36.5 Sec$^{-1}$ | 73 Sec$^{-1}$ |
| 100 | 950 ml | $8.75 \times 10^6$ | 4.1 | 3.55 | 2.76 | 2.42 |
| 12 | 1000 ml | $9.3 \times 10^7$ | 4.0 | 3.65 | 2.90 | 2.54 |
| 6 | 805 ml | $1.74 \times 10^8$ | 4.1 | 3.50 | 2.78 | 2.48 |
| 3 | 765 ml | $1.68 \times 10^8$ | 4.1 | 3.50 | 2.90 | 2.51 |
| 1.5 | 630 ml | $1.9 \times 10^8$ | 4.1 | 3.50 | 2.82 | 2.48 |
| Control | 305 ml | $2.0 \times 10^8$ | 4.1 | 3.50 | 2.80 | 2.50 |

a) 1.2 $\mu$ Millipore filter with a 1.4 Kg/cm² positive pressure.

**Claims**

1. A process for degrading water insoluble matter present in an aqueous polysaccharide solution derived from xanthomonas fermentation of carbohydrates, which comprises contacting the polysaccharide solution with a complex enzyme having cell-lytic, $\beta$-1,3-glucanase and protease activity, said enzyme being obtained from *Pellicularia filamentosa* and/or *Pellicularia sasakii.*

2. A process as claimed in claim 1, wherein the complex enzyme is obtained from *Pellicularia filamentosa var, sasakii* ATCC No. 23065.

3. A process as claimed in claim 1 or 2 wherein the complex enzyme is present in the aqueous solution in an amount from 10 to 100 ppm.

4. A process as claimed in claim 1, 2 or 3 wherein the contacting is carried out at a temperature of from 20° to 35°C.

5. A process as claimed in claim 1, 2, 3 or 4 wherein the contacting is carried out at a pH in the range of 2 to 10.

6. A process as claimed in any one of the preceding claims wherein the aqueous polysaccharide is contacted with from 600 to 250 cell wall-lysing units of complex enzyme per gram of xanthan gum.

7. A process as claimed in any one of the preceding claims wherein the aqueous polysaccharide solution contains from 0.8 to 0.5% by weight of xanthan gum.

8. A solid composition comprising 90 to 99.5% by weight xanthan gum; 0.3 to 0.5% by weight complex enzyme having cell-lytic, $\beta$-1,3-glucanase and protease activity, and 0 to 10% by weight of a solid extender or inert diluent; said complex enzyme being produced by *Pellicularia filamentosa* and/or *Pellicularia sasakii.*

9. A composition as claimed in claim 8 wherein the complex enzyme is produced by *Pellicularia filamentosa var. sasakii.* ATCC No. 20365.

**Revendications**

1. Un procédé pour dégrader la matière insoluble dans l'eau présente dans une solution aqueuse de polysaccharide dérivée de la fermentation par xanthomonas d'hydrates de carbone, qui comprend la mise en contact de la solution de polysaccharide avec une enzyme complexe ayant une activité de lyse de cellules, de bêta-1,3-glucanase et de protéase, cette enzyme étant obtenue à partir de *Pellicularia filamentosa* et/ou de *Pellicularia sasakii.*

2. Un procédé selon la revendication 1, dans lequel l'enzyme complexe est obtenue à partir de *Pellicularia filamentosa var. sasakii* ATCC N° 20365.

3. Un procédé selon l'une des revendications 1 et 2, dans lequel l'enzyme complexe est présente à raison de 10 à 100 ppm.

4. Un procédé selon l'une des revendications 1 à 3, dans lequel la mise en contact est effectuée à une température comprise entre 20°C et 35°C.

5. Un procédé selon l'un des revendications 1 à 4, dans lequel la mise en contact est effectuée à un pH compris entre 2 et 10.

6. Un procédé selon l'une quelconque des revendications précédentes, dans lequel le poly-

# 0 039 962

saccharide aqueux est mis en contact avec 600 à 250 unités de lyse de parois de cellules d'enzyme complexe par gramme de gomme xanthane.

7. Un procédé selon l'une quelconque des revendications précédentes, dans lequel la solution aqueuse de polysaccharide contient de 0,8 à 0,5% en poids de gomme xanthane.

8. Une composition solide comprenant de 90 à 99,5% en poids de gomme xanthane, de 0,3 à 0,5% en poids d'enzyme complexe ayant une activité de lyse de cellules, de bêta-1,3-glucanase et de protéase et de 0 à 10% en poids d'un diluant solide ou d'un diluant inerte; l'enzyme complexe étant produite par *Pellicularia filamentosa* et/ou *Pellicularia sasakii*.

9. Une composition selon la revendication 8, dans laquelle l'enzyme complexe est produite par *Pellicularia filamentosa var. sasakii* ATCC N° 20365.

## Patentansprüche

1. Verfahren zum Abbau wasserunlöslicher Substanzen in einer wäßrigen Polysaccharid-lösung, die gebildet worden ist durch Xanthomonas-Fermentation von Kohlenhydraten, umfassend das Zusammenbringen der Polysaccharidlösung mit einem komplexen Enzym mit die Zellen auflösender (zellytischer), $\beta$-1,3-Glucanase- und Protease-aktivität, wobei das Enzym erhalten worden ist von Pellicularia filamentosa und/oder Pellicularia sasakii.

2. Verfahren nach Anspruch 1, wobei das komplexe Enzym erhalten worden ist von Pellicularia filamentosa var. sasakii ATCC Nr. 20365.

3. Verfahren nach Anspruch 1 oder 2, wobei das komplexe Enzym in der wäßrigen Lösung in einer Menge von 10 bis 100 ppm vorhanden ist.

4. Verfahren nach Anspruch 1, 2 oder 3, wobei das Zusammenbringen bei einer Temperatur von 20 bis 35°C durchgeführt wird.

5. Verfahren nach Anspruch 1, 2, 3 oder 4, wobei das Zusammenbringen bei einem pH-Wert im Bereich von 2 bis 10 durchgeführt wird.

6. Verfahren nach einem der vorausgehenden Ansprüche, wobei das wäßrige Polysaccharid mit 600 bis 250 Zellwand auflösenden Einheiten des komplexen Enzymspro Gramm Xanthangummi zusammengebracht wird.

7. Verfahren nach einem der vorausgehenden Ansprüche, wobei die waßrige Polysaccharidlosung 0,8 bis 0,5 Gew-% Xanthangummi enthält.

8. Festes Mittel, umfassend 90 bis 99,5 Gew.-% Xanthangummi 0,3 bis 0,6 Gew.-% komplexes Enzym mit zellösender, $\beta$-1,3-Glucanase- und Protease-aktivität und 0 bis 10 Gew.-% eines festen Streckmittels oder inerten Verdünnungsmittels, wobei das komplexe Enzyme gebildet worden ist von Pellicularia filamentosa und/oder Pellicularia sasakii.

9. Mittel nach Anspruch 8, wobei das komplexe Enzym gebildet worden ist von Pellicularia filamentosa var. sasakii ATCC Nr. 20365.

7